# EUROPEAN PATENT APPLICATION

(11) **EP 4 335 304 A1**
(43) Date of publication of application: **13.03.2024**
(21) Application number: 22917640.9
(22) Date of filing: 05.12.2022
(51) Int. Cl.: A23L 33/10, A23L 29/00, C12N 1/16, C12N 1/20

(54) **FERMENTED APPLE COMPOSITION**

(30) Priority: 29.07.2022 KR 20220094865
(71) Applicant: CJ Cheiljedang Corporation, Seoul 04560 (KR)
(72) Inventor: HONG, Na Youn, Jung-gu, Seoul 04560 (KR); JUNG, Hee Kyoung, Jung-gu, Seoul 04560 (KR); KIM, Sunhee, Jung-gu, Seoul 04560 (KR); PARK, Yeeun, Jung-gu, Seoul 04560 (KR); LEE, Eunyong, Jung-gu, Seoul 04560 (KR)
(74) Representative: Icosa
(86) International application number: PCT/KR2022/019572
(87) International publication number: WO 2024/025051

(57) **Abstract**

Provided are a fermented apple composition having an effect of reducing or inhibiting expression of inflammatory cytokines, and use thereof in the prevention or improvement of enteritis.

## Description

### [Technical Field]

The present disclosure relates to a fermented apple composition and use thereof in the prevention or improvement of enteritis.

### [Background Art]

The intestine, which is the main place where substances introduced from the outside come into contact, plays an important role in protecting our body from various external stimuli. Intestinal defense is primarily based on the structural features and intrinsic motility of the intestinal tract, and the defense mechanism acts secondary by cellular and humoral immunity related to the permeability of intestinal epithelial cells.

The tight junctions that exist between cells affect the permeability of intestinal epithelial cells. When tight junctions loosen due to stimuli or damage in the intestine, harmful molecules (pathogens, toxins, antigens, etc.) excessively permeate the intestinal epithelium, causing endotoxemia, disrupting the mucosal immune system and inducing an inflammatory response, leading to bowel diseases or systemic diseases with inflammation.

Inflammatory bowel disease (IBD), which shows a recent increasing trend of incidence among bowel diseases, is a disease caused by neutrophils as a mediator. These neutrophils are attracted by interleukin-8 (IL-8), which is increased in inflamed mucosa (Non-Patent Document 0001). IL-8 is one of inflammatory cytokines, involved in tumorigenesis and angiogenesis in addition to attracting neutrophils, and plays an important role in various pathological conditions such as chronic inflammation and cancer (Non-Patent Document 0002). For example, various types of cells, including macrophages and tumor cells, secrete IL-8 as an inflammatory response induced by lipopolysaccharide (LPS) stimulation.

Meanwhile, apples are fruits of the apple tree, which is a perennial plant belonging to the genus *Malus,* the family *Rosaceae,* and they have a high content of dietary fiber such as pectin, organic acid, sugar, vitamins and inorganic salts. In particular, pectin, which is a water-soluble dietary fiber contained in large amounts in apples, is effective for constipation by helping intestinal movement, and organic acids are well known to help digestion and absorption by promoting secretion of gastric juice.

Currently, a number of various functional foods and health supplements for improving intestinal health using apples have been developed. However, with increasing domestic and foreign demand for compositions capable of improving intestinal health by preventing or improving intestinal inflammation, the development of a new composition having more excellent effects on preventing or improving intestinal inflammation is required.

### [Disclosure]

### [Technical Problem]

The present disclosure provides a fermented apple composition and use thereof in the prevention or improvement of enteritis.

### [Technical Solution]

An object of the present disclosure is to provide a fermented apple composition having an effect of reducing or inhibiting expression of inflammatory cytokines.

Another object of the present disclosure is to provide a food including the fermented apple composition of the present disclosure.

Still another object of the present disclosure is to provide a health functional food including the fermented apple composition of the present disclosure.

### [Advantageous Effects]

Since a fermented apple composition of the present disclosure has an effect of reducing or inhibiting the expression of inflammatory cytokines, it may be provided for use in preventing or improving enteritis.

### [Brief Description of the Drawings]

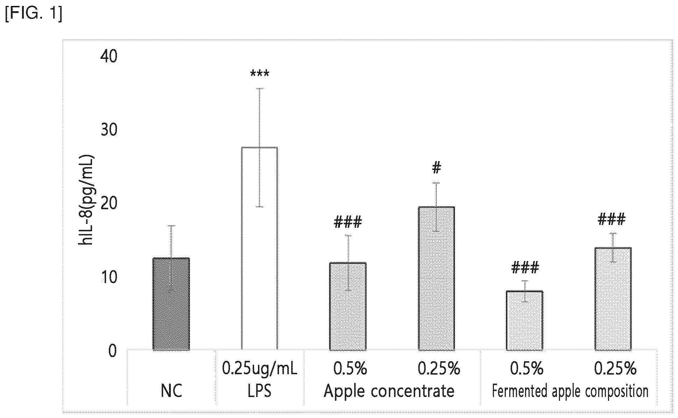

FIG. 1 shows results of examining expression levels of human interleukin-8 (hIL-8) by concentration by treating HT-29 cells with different concentrations of a fermented apple composition or an apple concentrate together with lipopolysaccharide (LPS). ***p < 0.001 (vs NO group), ###p < 0.001, #p < 0.05 (vs LPS group).

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a fermented apple composition having an effect of reducing or inhibiting expression of inflammatory cytokines.

The fermented apple composition of the present disclosure may include purified water and an apple concentrate.

As used herein, the term "apple concentrate" means a liquid obtained by concentrating apples, and those prepared or those commercially available may be used without limitation. For example, the apple concentrate may be a 100% apple concentrate, but is not limited thereto. Further, the apple concentrate may have a sugar content of 60 brix or more, based on a refractometer, but is not limited thereto. In addition, the apple concentrate may have an acidity of 6% or less, but is not limited thereto.

In one embodiment, the apple concentrate in the fermented apple composition of the present disclosure may refer to a component included before fermentation. Specifically, in the fermented apple composition of the present disclosure, the apple concentrate may be included in a volume of 1%(v/v) to 50%(v/v), 1%(v/v) to 30%(v/v), 1%(v/v) to 25%(v/v), 5%(v/v) to 50%(v/v), 5%(v/v) to 30%(v/v), 5%(v/v) to 25%(v/v), 10%(v/v) to 50%(v/v), 10%(v/v) to 30%(v/v), 10%(v/v) to 25%(v/v), 15%(v/v) to 50%(v/v), 15%(v/v) to 30%(v/v) or 15%(v/v) to 25%(v/v) with respect to the total volume of the fermented apple composition.

As used herein, the term "fermented apple composition" refers to a fermentation product resulting from fermentation of the apple concentrate.

As used herein, the term "fermentation" commonly refers to a phenomenon or process in which microorganisms convert and accumulate metabolites by decomposing organic matter using their own enzymes.

As used herein, the term "fermentation product" may be comprehensively interpreted as including all of a material produced by fermentation of the present disclosure, a filtrate thereof, a dilution thereof, a concentrate thereof, a crude product thereof, or a purified product thereof, etc., but is not limited thereto.

Specifically, the fermentation product of the present disclosure may be an acetic acid fermentation product or vinegar. More specifically, the fermentation product of the present disclosure may be a yeast and acetic acid bacteria fermentation product. Much more specifically, the fermentation product of the present disclosure may be a fermentation product of acetic acid bacteria after yeast fermentation.

For example, the fermented apple composition of the present disclosure may be an apple acetic acid-fermented composition, an apple acetic acid fermentation product, or an apple vinegar, which is obtained by fermentation of apple with acetic acid bacteria. Further, the fermented apple composition of the present disclosure may be an apple yeast-fermented and acetic acid bacteria-fermented composition, which is obtained by fermentation of apple with yeast and acetic acid bacteria. Furthermore, the fermented apple composition of the present disclosure may be an acetic acid bacteria-fermented composition post-yeast fermentation of apple, which is obtained by fermentation of apple with acetic acid bacteria after fermentation of apple with yeast.

The inflammatory cytokine of the present disclosure may be interleukin-8 (IL-8), IL-1α, IL-12, tumor necrosis factor alpha (TNF-α), or interferon gamma (IFN-γ), and any inflammatory cytokine may be included in the scope of the present disclosure, as long as it is known in the art. In one embodiment, the inflammatory cytokine may be IL-8.

When inflammation-induced cells are treated with the fermented apple composition, the inflammation-induced inflammatory cytokine expression may be reduced or inhibited, as compared to inflammation-induced cells without treatment with the fermented apple composition.

For example, the fermented apple composition may reduce or inhibit the inflammation-induced inflammatory cytokine expression in the range consisting of one lower limit selected from 10% or more, 15% or more, 20% or more, 25% or more, 30% or more, 35% or more, 40% or more, 45% or more, 46% or more, 50% or more, 54% or more, 55% or more, 60% or more, 65% or more, and 69% or more, and/or one upper limit selected from 100% or less, 90% or less, 80% or less, and 70% or less, but is not limited thereto.

In the present disclosure, the fermented apple composition of the present disclosure may have an anti-inflammatory effect.

In the present disclosure, the fermented apple composition of the present disclosure may have an effect of preventing or improving enteritis.

As used herein, the term "enteritis" refers to inflammation that occurs in the intestine, and refers to inflammation caused by stimulation of the intestine due to viruses, bacteria, ingestion of toxins, etc.

As used herein, the term "preventing" means all of the actions by which enteritis is restrained or retarded by the administration of the composition of the present disclosure.

As used herein, the term "improving" means all of the actions by which symptoms in an individual who is suspected of having enteritis and has developed enteritis have taken a turn for the better or been modified favorably by the composition.

The anti-inflammation or the prevention or improvement of enteritis may be achieved by, for example, reducing or inhibiting expression of inflammatory cytokines, which is as described above.

In the present disclosure, the fermented apple composition of the present disclosure may have an effect of improving or enhancing intestinal health.

The improvement or enhancement of intestinal health may be achieved by, for example, reducing or inhibiting expression of inflammatory cytokines, which is as described above.

Another aspect of the present disclosure provides a food including the fermented apple composition of the present disclosure.

The fermented apple composition is as described above.

In the present disclosure, the food of the present disclosure may have an effect of preventing or improving enteritis. The prevention or improvement of enteritis may be achieved by, for example, reducing or inhibiting expression of inflammatory cytokines, which is as described above.

In the present disclosure, the food of the present disclosure may have an effect of improving or enhancing intestinal health. The improvement or enhancement of intestinal health may be achieved by, for example, reducing or inhibiting expression of inflammatory cytokines, which is as described above.

Still another aspect of the present disclosure provides a health functional food including the fermented apple composition of the present disclosure.

The fermented apple composition is as described above.

In the present disclosure, the health functional food of the present disclosure may have an effect of preventing or improving enteritis. The prevention or improvement of enteritis may be achieved by, for example, reducing or inhibiting expression of inflammatory cytokines, which is as described in the previous aspect.

In the present disclosure, the health functional food of the present disclosure may have an effect of improving or enhancing intestinal health. The improvement or enhancement of intestinal health may be achieved by, for example, reducing or inhibiting expression of inflammatory cytokines, which is as described in the previous aspect.

The "food" of the present disclosure may include all types of general foods, functional foods, nutritional supplements, health foods, food additives, etc.

The above type of food may be prepared into various forms according to common methods known in the art.

The food includes forms such as pills, powders, granules, infusions, tablets, capsules, powders or liquids, etc., and foods to which the composition of the present disclosure may be added may include, for example, various foods, such as rice, edible grain flour (edible grain powder), grain soup, rice bowl, noodles, rice soup, instant rice, seasoning, lunch box rice, dried cooked rice, bread, edible sugar, rice cake, bibimjang, sauce, spice, edible salt, seasoning, seasoning powders, processed, frozen, dried and cooked fruits and vegetables, jellies, jams, compotes, eggs, milk and other dairy products, edible oils and fats, coffee, cocoa and coffee substitutes, tapioca, grain flour and grain preparations, ramen, udon, noodles, noodle soup, cold noodles, porridge, soup, soup dishes, instant foods, frozen foods, instant foods, retort foods, other beverages, chewing gum, tea, vitamin complex, health supplements, etc., but is not limited thereto.

As ingredients that may be included in the food of the present disclosure, various herbal extracts, food auxiliary additives, or natural carbohydrates may be included as additional ingredients, like in general foods. Further, the food auxiliary additives may include food auxiliary additives common in the art, for example, flavoring agents, savory agents, coloring agents, fillers, stabilizers, etc.

Examples of the natural carbohydrates include monosaccharides, for example, glucose, fructose, etc.; disaccharides, for example, maltose, sucrose, etc.; and polysaccharides, for example, common sugars such as dextrins, cyclodextrins, etc., and sugar alcohols such as xylitol, sorbitol, erythritol, etc. In addition to those described above, natural flavors (e.g., rebaudioside A, glycyrrhizin, etc.) and synthetic flavors (saccharin, aspartame, etc.) may be advantageously used as flavoring agents. In addition, common food additives used for the purpose of supplementing taste and nutrition, for example, nucleic acids, amino acids, organic acids, etc., may be added.

In addition to those described above, the food of the present disclosure may include various nutrients, vitamins, minerals (electrolytes), flavors such as synthetic flavors and natural flavors, etc., coloring agents and fillers (cheese, chocolate, etc.), pectic acid or salts thereof, alginic acid and salts thereof, organic acids, protective colloidal thickeners, pH adjusters, stabilizers, preservatives, glycerin, alcohols, carbonation agents used in carbonated drinks, etc. In addition, the food may include fruit flesh for the preparation of natural fruit juices, fruit juice beverages and vegetable beverages. These components may be used independently or in combination.

The food of the present disclosure may be prepared by a method commonly used in the art, and may be prepared by adding raw materials and ingredients commonly added in the art during the preparation. In addition, the formulation of the food may also be prepared without limitation as long as the formulation is recognized as a food.

Further, when the food of the present disclosure is used as a health functional food, the food of the present disclosure may be prepared in various types of formulations, and unlike general medicines, since the food is used as a raw material, there are advantages of being free from side effects that may occur when taken for a long period of time, and of being highly portable, and therefore, the food of the present disclosure may be ingested as a supplement.

Meanwhile, the food of the present disclosure may also include flavoring agents, savory agents, coloring agents, fillers, stabilizers, and flavors which may also be classified as food additives.

As used herein, the term 'flavor' may be a material added to enhance the flavor of food. The flavor may be a material that allows foods to have excellent seasoning property.

The flavor may be divided according to taste components. In other words, according to the taste, the flavor may be divided into neutral flavor, beef flavor, chicken flavor, pork flavor, kokumi flavor, etc.

The 'kokumi flavor' means a flavor which releases the flavor of kokumi, and the 'kokumi' is a Japanese word, and may also be expressed as 'mouthfulness', 'continuity', 'thickness', and 'heartiness' in English, and expressed as 'rich taste', 'thick taste', 'mouth-filling taste', 'dense taste', 'sticky taste', etc. in Korean. The 'neutral flavor' refers to a flavor that maximizes `umami' and minimizes other flavors to produce a mild and clean flavor. For example, oils such as canola oil or grapeseed oil among oils may be acknowledged as having a neutral flavor. The term 'seasoning property' may mean to have a sour, sweet, salty, bitter, or umami taste, but is not limited thereto.

The food of the present disclosure may be a CJ CheilJedang Co., Ltd.'s apple vinegar or fruit fermented cider vinegar, for example, Petitzel Micho^{®} or Micho^{®} product, but is not limited thereto. The food of the present disclosure may include the fermented apple composition of the present disclosure at a concentration of 1 %(v/v) to 50%(v/v), 1 %(v/v) to 30%(v/v), 5%(v/v) to 50%(v/v), 5%(v/v) to 30%(v/v) or 10%(v/v) to 30%(v/v) with respect to the total volume of the food, but is not limited thereto.

Still another aspect of the present disclosure provides a method of reducing or inhibiting expression of inflammatory cytokines, the method including the step of administering the fermented apple composition of the present disclosure.

Still another aspect of the present disclosure provides a method of preventing, improving, or treating enteritis, the method including the step of administering the fermented apple composition of the present disclosure.

As used herein, the term "administering" means introducing the composition of the present disclosure to a subject, in which inflammatory cytokines are excessively expressed or enteritis is suspected, by any suitable method.

The pharmaceutical composition of the present disclosure is not particularly limited, as long as the subject is a subject for the purpose of reducing or inhibiting the expression of inflammatory cytokines, or for the purpose of preventing, improving, or treating enteritis, and the composition is applicable to any subject. For example, the composition may be applied to any subject of non-human animals such as monkeys, dogs, cats, rabbits, guinea pigs, rats, mice, cows, sheep, pigs, goats, etc., birds and fish, etc.

The composition of the present disclosure may be administered in an amount effective for reducing or inhibiting the expression of inflammatory cytokines, or may be administered in an amount effective for preventing, improving, or treating enteritis. The preferred dosage of the composition of the present disclosure may vary depending on the subject's conditions and body weight, severity of the disease, the type of drug, the route of administration and the duration, but may be appropriately selected by those skilled in the art.

In addition, the composition of the present disclosure may be administered through various oral or parenteral routes as long as it is able to reach a target tissue. For example, it may be administered orally, parenterally, subcutaneously, intraperitoneally, intrapulmonary, intranasally, rectally or intravenously, intramuscularly, subcutaneously, intrauterine or intracerebral injection, and for local treatment, if necessary, by any suitable method including intralesional administration, but is not limited thereto.

In one embodiment, the fermented apple composition may be administered in the form of a food composition including the same. In this regard, the administration may be oral administration.

In another embodiment, the fermented apple composition may be administered in the form of a health functional food composition including the same. In this regard, the administration may be oral administration.

Still another aspect of the present disclosure provides use of the fermented apple composition of the present disclosure in reducing or inhibiting the expression of inflammatory cytokines.

The fermented apple composition may reduce or inhibit the inflammation-induced inflammatory cytokine expression by 10% or more when inflammation-induced cells are treated therewith, as compared to inflammation-induced cells without treatment with the fermented apple composition, but is not limited thereto. This is as described above.

The inflammatory cytokine may be IL-8, but is not limited thereto. This is as described above.

Still another aspect of the present disclosure provides anti-inflammatory use of the fermented apple composition of the present disclosure.

Still another aspect of the present disclosure provides use of the food including the fermented apple composition of the present disclosure in preventing, treating, or improving enteritis.

Still another aspect of the present disclosure provides use of the food including the fermented apple composition of the present disclosure in improving or enhancing intestinal health.

Still another aspect of the present disclosure provides use of the health functional food including the fermented apple composition of the present disclosure in preventing, treating, or improving enteritis.

Still another aspect of the present disclosure provides use of the health functional food including the fermented apple composition of the present disclosure in improving or enhancing intestinal health.

The terms used herein are as described in the previous aspects.

### [Mode for Carrying Out the Invention]

Hereinafter, the present disclosure will be described in more detail with reference to exemplary embodiments. However, the following exemplary embodiments are merely preferred embodiments for illustrating the present disclosure, and therefore, are not intended to limit the scope of the present disclosure thereto. On the other hand, technical details not described in this specification may be sufficiently understood and easily implemented by those skilled in the art or similar art.

### Preparation Example 1: Preparation of fermented apple composition

An apple concentrate was a commercially available product with 100% concentration of apples. An apple concentrate made in China, of which a sugar content was 60 brix or more, based on a refractometer, and an acidity was 6% or less, was diluted with distilled water 4 times (w/w) to prepare the apple concentrate at about 20 brix.

As a fermented apple composition, a commercially available apple cider vinegar (a yeast and acetic acid bacteria-fermented product, containing purified water and 20.86% of apple concentrate and having acidity of 6% to 8%; healthy fermented apple cider vinegar from CJ CheilJedang Co., Ltd.) was used.

To remove microorganisms from the fermented apple composition and the apple concentrate, filtration was performed using a 0.2 um filter.

### Example 1: Evaluation of anti-inflammatory ability in intestine

The anti-inflammatory ability of the fermented apple composition in the intestine was analyzed by expression levels of human interleukin-8 (hIL-8) in HT-29 cells, in which inflammation was induced by lipopolysaccharide (LPS), according to the method of Lee et al. (Lee et al., Biochem Biophys Res Commun 337(2), Nov 2005, pages 457 - 463).

In detail, the HT-29 cell line (Korea Cell Line Bank) was cultured in an RPMI medium (Gibco, USA) supplemented with heat-inactivated 10% fetal bovine serum (FBS), 1% L-glutamine, penicillin G (100 IU/mL) and streptomycin (100 mg/mL) under conditions of 37°C and 5% CO₂. The cultured HT-29 cells were dispensed into a 96-well plate (Corning, USA) at a density of 1.5 x 10⁴ cells/well (volume of 250 µL), and cultured for 3 days until a monolayer was completely formed (FIG. 1, indicated by NC). Then, the supernatant was removed. As an experimental group, each 250 uL/wells of the fermented apple composition (0.25% (v/v), 0.5% (v/v)) and the apple concentrate (0.25% (v/v), 0.5% (v/v)) of Preparation Example 1 was treated together with 0.25 ug/mL of LPS, followed by incubation for 16 hours. An untreated group was treated with only 0.25 ug/mL of LPS, followed by incubation (FIG. 1, indicated by LPS). Although pH of the fermented apple composition was slightly low, the sample dilution factor was quite high, and the pH did not change due to the buffering effect after dilution with the cell medium. Accordingly, pH adjustment was not performed. After culturing, the supernatant was taken and centrifuged at 4°C at 13,000 rpm for 3 minutes to obtain only the supernatant excluding cells. Thereafter, to remove residual cells in the supernatant, filtration was performed using a 0.2 um filter.

The expression levels of hIL-8 were measured by analyzing the obtained supernatant by an ELISA assay.

In detail, the ELISA assay was performed according to the instructions of the manufacturer of a Duoset ELISA ancillary reagent kit 2 (R&D systems, cat DY008, USA) as follows. To perform the ELISA assay, a human IL-8/CXCL8 Quantikine ELISA Kit (R&D systems, cat number D8000C, USA) containing an IL-8 antibody and a biotinylated secondary antibody was used. An IL-8 capture antibody (R&D systems, cat number 890462) was attached to each well of the ELISA plate at room temperature for 24 hours. The supernatant of each experimental group and control group and IL-8 standard solution (R&D systems, cat number 890466) were diluted with a reaction diluent (R&D systems, cat number 895877), and 100 µL thereof was added to each well, followed by incubation at room temperature for 2 hours. Thereafter, the culture medium was removed, and a secondary antibody, biotinylated human IL-8 antibody (R&D systems, cat number 890465) was added and allowed to attach at room temperature for 2 hours. The color development was induced by treating each well with streptavidin-conjugated horseradish peroxidase (R&D systems, cat number 893975) and a substrate thereof, tetramethylbenzidine (TMB, R&D systems, cat number DY999). At this time, color development proceeded from blue to yellow. The reaction was stopped by treating with 50 µL of a reaction stopping solution (R&D systems, cat number 895032), and then absorbance was measured at 470 nm (correction value at 540 nm) using a spectrophotometer.

As a result, both the fermented apple composition-treated group and the apple concentrate-treated group showed a significant reduction in the hIL-8 expression level at concentrations of 0.25% (v/v) and 0.5% (v/v) with no cytotoxicity, as compared to the control group (LPS) treated only with LPS (Table 1, FIG. 1, ###p < 0.001, #p < 0.05).

In addition, when comparing the anti-inflammatory ability in the intestine at the same concentrations, the reduction rate of hIL-8 expression level was higher in the fermented apple composition (46% at 0.25%, 69% at 0.5%) than in the apple concentrate (25% at 0.25%, 54% at 0.5%) (Table 1, FIG. 1, ###p < 0.001, #p < 0.05).

Accordingly, it was confirmed that both the fermented apple composition and the apple concentrate exhibited anti-inflammatory effects in the intestine, and in particular, the fermented apple composition had the excellent anti-inflammatory effect, as compared to the apple concentrate.

**[Table 1]**

| **Samples** | | **Average** | **Stdev** | **T-test (NO)** | | **T-test (LPS)** | |
|---|---|---|---|---|---|---|---|
| NC | | 27.68125 | 4.3550572 | 1 | | 4.479E-11 | *** |
| LPS | 0.25ug/mL | 57.644444 | 8.0511022 | 4.479E-11 | *** | 1 | |
| Apple concentrate | 0.5%(v/v) | 26.266667 | 3.7383894 | 0.624301 | | 0.0001409 | *** |
| | 0.25%(v/v) | 43.066667 | 3.2967998 | 3.904E-05 | *** | 0.0192483 | * |
| Fermented apple composition | 0.5%(v/v) | 17.666667 | 1.4383633 | 0.0016675 | ** | 1.453E-05 | *** |
| | 0.25%(v/v) | 30.8 | 1.9510681 | 0.2653488 | | 0.0003893 | *** |

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A fermented apple composition having an effect of reducing or inhibiting expression of inflammatory cytokines.

2. The fermented apple composition of claim 1, wherein the fermented apple composition reduces or inhibits the inflammation-induced inflammatory cytokine expression by 10% or more when inflammation-induced cells are treated therewith, as compared to inflammation-induced cells without treatment with the fermented apple composition.

3. The fermented apple composition of claim 1, wherein the inflammatory cytokine is IL-8.

4. The fermented apple composition of claim 1, wherein the fermented apple composition has an anti-inflammatory effect.

5. The fermented apple composition of claim 1, wherein the fermented apple composition is an apple acetic acid-fermented composition.

6. The fermented apple composition of claim 5, wherein the fermented apple composition is an apple yeast-fermented and acetic acid bacteria-fermented composition.

7. A food comprising the fermented apple composition of claim 1.

8. The food of claim 7, wherein the food is for preventing or improving enteritis.

9. The food of claim 7, wherein the food is for improving or enhancing intestinal health.

10. A health functional food comprising the fermented apple composition of claim 1.

11. The health functional food of claim 10, wherein the health functional food is for preventing or improving enteritis.

12. The health functional food of claim 10, wherein the health functional food is for improving or enhancing intestinal health.
